# EUROPÄISCHE PATENTANMELDUNG

(11) **EP 0 992 227 A2**
(43) Veröffentlichungstag der Anmeldung: **12.04.2000**
(21) Anmeldenummer: 99116931.9
(22) Anmeldetag: 26.08.1999
(51) Int. Cl.: A61G 13/10, F16B 7/04

(54) **Vorrichtung zum einstellbaren Verbinden von zwei Gegenständen**

(30) Priorität: 05.10.1998 DE 19845625
(71) Anmelder: pro med instruments GmbH, Herstellung und Vertrieb medizinisch technischer Ausrüstung, 79111 Freiburg (DE)
(72) Erfinder: Kiefer, Joachim, 79100 Freiburg (DE)
(74) Vertreter: RACKETTE Partnerschaft Patentanwälte

(57) **Zusammenfassung**

Eine Vorrichtung zum einstellbaren Verbinden von zwei Gegenständen verfügt über einen Handgriff (1) und einen Anlenkhebel (2). Über an dem Handgriff (1) und dem Anlenkhebel (2) ausgebildete lösbare Gelenkmittel ist eine Betätigungsstange (22) zum Verengen von Klemmspalten (8, 10) bei Überführung des Anlenkhebels (2) von einer ersten Stellung in eine zweite Stellung betätigbar. An dem Handgriff (1) und dem Anlenkhebel (2) sind Führungsmittel (16, 30, 31, 32, 33) ausgebildet, mit denen die Gelenkmittel in einer Einführendstellung des Anlenkhebels (2) in bezug auf den Handgriff (1) definiert miteinander in Eingriff bringbar sind. Dadurch ist eine ordnungsgemäße Verbindung der Gelenkmittel unter Vermeidung von Beschädigungen durch unsachgemäße Handhabung erzielt.

## Beschreibung

Die Erfindung betrifft eine Vorrichtung zum einstellbaren Verbinden von zwei Gegenständen, insbesondere einer chirurgischen Kopfhalterung mit Anbauteilen an einem Operationstisch.

Aus der EP 0 660 694 B1 ist eine Vorrichtung zum einstellbaren Verbinden einer chirurgischen Kopfhalterung mit Anbauteilen an einem Operationstisch als Gegenstände bekannt. Diese Vorrichtung ist durch zwei einander gegenüberliegende Armteile gebildet, die parallel zueinander ausgerichtet sind. An ihren Enden weisen die Armteile jeweils Ausnehmungen auf, durch die jeweils eine Klemmschraube durchführbar ist. Zum Verbinden der Kopfhalterung mit den Anbauteilen wird an jedem Endbereich der Vorrichtung zwischen die Armteile ein Rahmenteil der Kopfhalterung beziehungsweise der Anbauteile eingefügt und durch Anzug der Klemmschrauben verklemmt. Mit dieser Vorrichtung ist zwar die Kopfhalterung mit den Anbauteilen einstellbar miteinander verbindbar, allerdings ist die Handhabung durch die erforderliche Betätigung von zwei Klemmschrauben beispielsweise insbesondere bei unter gewissen Streßbedingungen erfolgenden chirurgischen Eingriffen im Kopfbereich in nachteiliger Weise verhältnismäßig umständlich.

Aus der Praxis sind Vorrichtungen zum einstellbaren Verbinden von zwei Gegenständen, insbesondere einer chirurgischen Kopfhalterung mit Anbauteilen an einem Operationstisch, mit einem Handgriff und einem Anlenkhebel bekannt, die über lösbare Gelenkmittel verfügen. Der Anlenkhebel ist mit einer Betätigungsstange verbunden, mittels der in Ausnehmungen des Handgriffs eingefügte Gegenstände unter Verklemmung mit dem Handgriff untereinander verbindbar sind. Bei diesen Vorrichtungen besteht jedoch der erhebliche Nachteil, daß bei unsachgemäßem Zusammenfügen des Handgriffs sowie des Anlenkhebels die Gelenkmittel nur schwer erkennbar nicht ordnungsgemäß miteinander in Eingriff stehen und bei Betätigung des Anlenkhebels beispielsweise bei chirurgischen Eingriffen nicht nur eine unter Umständen gefährliche Fehlbedienung der Vorrichtung erfolgt, sondern auch in nachteiliger Weise irreparable Beschädigungen an den Gelenkmitteln auftreten können.

Der Erfindung liegt die Aufgabe zugrunde, eine Vorrichtung zum einstellbaren Verbinden von zwei Gegenständen, insbesondere einer chirurgischen Kopfhalterung mit Anbauteilen an einem Operationstisch, anzugeben, die ein zuverlässiges Zusammenführen von einen Handgriff und einen Anlenkhebel der Vorrichtung verbindenden, lösbaren Gelenkmittel und damit eine ordnungsgemäße Funktionsweise der Vorrichtung auch ohne vorherige umständliche Kontrolle sicherstellt.

Diese Aufgabe wird gelöst mit einer Vorrichtung zum einstellbaren Verbinden von zwei Gegenständen, insbesondere einer chirurgischen Kopfhalterung mit Anbauteilen an einem Operationstisch, mit einem länglichen Handgriff, in dessen beiden Endbereichen jeweils eine Ausnehmung zum Einführen von miteinander zu verbindenden Gegenständen vorgesehen ist, mit einem Anlenkhebel, der an einem Ende mit einem Ende einer Betätigungsstange verbindbar ist, wobei das andere Ende der Betätigungsstange an dem Handgriff gegenlagerbar ist, mit an dem Handgriff sowie an dem Anlenkhebel ausgebildeten, voneinander lösbaren Gelenkmitteln, mit denen bei Überführung des Anlenkhebels von einer ersten Stellung in eine zweite Stellung auf die Betätigungsstange eine Kraft ausübbar ist, mittels der im Bereich der Ausnehmungen ausgebildete Klemmspalte unter Verklemmung von in die Ausnehmungen eingefügten Gegenständen verengbar sind, und mit an dem Handgriff sowie dem Anlenkhebel ausgebildeten Führungsmitteln, mit denen die Gelenkmittel in einer Einführstellung des Anlenkhebels in bezug auf den Handgriff definiert miteinander in Eingriff bringbar sind.

Dadurch, daß Führungsmittel vorgesehen sind, mit denen die Gelenkmittel in einer Einführstellung des Anlenkhebels in bezug auf den Handgriff definiert miteinander in Eingriff bringbar sind, ist auch ohne explizite Kontrolle die ordnungsgemäße Funktionsweise der Vorrichtung sicherstellt, da ein unsachgemäßes Zusammenführen des Handgriffs und des Anlenkhebels mit einer fehlerhaften Anordnung der Gelenkmittel ausgeschlossen ist.

Zweckmäßigerweise umfassen in einer Ausgestaltung der erfindungsgemäßen Vorrichtung die Führungsmittel wenigstens zwei paarweise einander gegenüberliegend ausgebildete Führungsvorsprünge, die an Seitenwänden einer Hebelausnehmung des Handgriffs angeordnet sind. Bei einer diesbezüglichen Weiterbildung ist es zweckmäßig, daß die Führungsvorsprünge als sich in Längsrichtung des Handgriffs erstreckende längliche Zapfen ausgebildet sind.

Bei der oben genannten Ausgestaltung sowie der diesbezüglichen Weiterbildung der erfindungsgemäßen Vorrichtung kann weiterhin vorteilhafterweise vorgesehen sein, daß der Abstand der paarweise einander gegenüberliegenden, an verschiedenen Seitenwänden angeordneten Führungsvorsprüngen im wesentlichen der Dicke des Anlenkhebels entspricht.

Bei der oben genannten Ausgestaltung sowie den diesbezüglichen Weiterbildungen kann weiterhin zweckmäßigerweise vorgesehen sein, daß die Führungsmittel weiterhin wenigstens ein Paar von einander gegenüberliegenden, jeweils an einer Seitenfläche des Anlenkhebels ausgebildete Begrenzungsvorsprünge umfassen, deren Abstand der Breite der Führungsvorsprünge quer zur Längsrichtung des Handgriffs entspricht oder größer als diese Breite ist. Hierbei ist es zweckmäßig, daß jeder Begrenzungsvorsprung als sich in Querrichtung des Anlenkhebels länglich erstreckender Zapfen ausgebildet ist und/oder daß der Abstand zwischen einander gegenüberliegenden, an verschiedenen Seitenflächen angeordneten Begrenzungsvorsprüngen der Weite der Hebelausnehmung entspricht.

Die vorgenannten Maßnahmen führen einzeln oder in Kombination zu einer Verbesserung der Funktionsweise der Führungsmittel.

Bei der erstgenannten zweckmäßigen Ausgestaltung der erfindungsgemäßen Vorrichtung sowie den entsprechenden Weiterbildungen ist es weiterhin zweckmäßig, daß die Gelenkmittel zwei einander gegenüberliegende, in der Hebelausnehmung innenseitig der Führungsvorsprünge angeordnete Anlenkzapfen umfassen. Zusätzlich kann vorgesehen sein, daß die Gelenkmittel eine in der ersten Stellung mit den Anlenkzapfen in Eingriff stehende, an dem Anlenkhebel ausgebildete Anlenknase umfassen. Auf diese Weise sind konstruktiv verhältnismäßig unkomplizierte und einfach zu handhabende Gelenkmittel geschaffen.

Im Hinblick auf eine zweckmäßige Verbinden des Anlenkhebels sowie des Handgriffs mit einer Betätigungsstange ist es zweckmäßig, daß der Handgriff eine Betätigungsstangenausnehmung aufweist, in der die Betätigungsstange positionierbar ist und daß in einer diesbezüglichen Weiterbildung an dem den Gelenkmitteln gegenüberliegenden Endbereich des Handgriffs ein Mündungsbereich der Betätigungsstangenausnehmung kalottenförmig ausgebildet ist, so daß darin ein abschnittsweise komplementär ausgebildeter Kopf einer auf die Betätigungsstange aufschraubbarer Widerlagerschraube drehbar gelagert ist.

Weitere zweckmäßige Ausgestaltungen sind Gegenstand der Unteransprüche sowie der nachfolgenden Beschreibung eines bevorzugten Ausführungsbeispiels der Erfindung unter Bezug auf die Figuren der Zeichnung. Es zeigen:
- Fig. 1: in einer perspektivischen Ansicht ein Ausführungsbeispiel einer erfindungsgemäßen Vorrichtung mit einem Handgriff und einem Anlenkhebel in demontierter Anordnung,
- Fig. 2: in einer teilgeschnittenen perspektivischen Ansicht das Ausführungsbeispiel gemäß Fig. 1 mit dem Handgriff und dem Anlenkhebel in einer Einführstellung,
- Fig. 3: in einer teilgeschnittenen Seitenansicht das Ausführungsbeispiel gemäß Fig. 1 mit dem Handgriff und dem Anlenkhebel in der Einführstellung gemäß Fig. 2,
- Fig. 4: in einer teilgeschnittenen perspektivischen Ansicht das Ausführungsbeispiel gemäß Fig. 1 mit dem Handgriff und dem Anlenkhebel in einer Verriegelungsstellung und
- Fig. 5: in einer teilgeschnittenen Seitenansicht das Ausführungsbeispiel gemäß Fig. 1 mit dem Handgriff und dem Anlenkhebel in der Verriegelungsstellung gemäß Fig. 4.

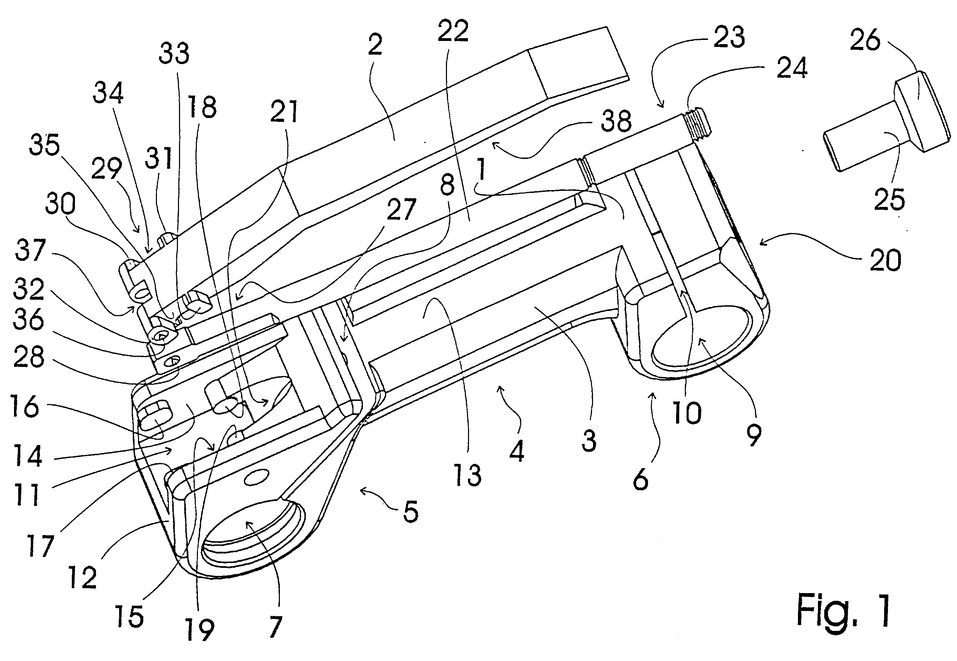

Fig. 1 zeigt in einer perspektivischen Ansicht ein Ausführungsbeispiel einer erfindungsgemäßen Vorrichtung mit einem Handgriff 1 und einem Anlenkhebel 2 in demontierter Anordnung. Der längliche Handgriff 1 verfügt über einen Mittelteil 3, an dem eine Griffmulde 4 ausgebildet ist. An das Mittelteil 3 des Handgriffs 1 schließen sich endseitig ein erster Endbereich 5 sowie ein zweiter Endbereich 6 an, die gegenüber dem Mittelteil 3 verdickt sind. In die Endbereiche 5, 6 sind jeweils eine erste Stangenausnehmung 7 sowie ein erster Klemmspalt 8 beziehungsweise eine zweite Stangenausnehmung 9 sowie ein zweiter Klemmspalt 10 eingebracht. In die Stangenausnehmungen 7, 9 mit vorzugsweise rundlichem Querschnitt sind zu verbindende Gegenstände wie beispielsweise jeweils eine Verbindungsstange einer chirurgischen Kopfhalterung und von Anbauteilen an einem Operationstisch, die in Fig. 1 nicht dargestellt sind, einfügbar, wobei bei Verengung der sich von der Innenseite der Stangenausnehmungen 7, 9 bis zu einer Außenseite des Handgriffs 1 erstreckenden Klemmspalte 8, 10 in einer weiter unten näher erläuterten Weise die Gegenstände einklemmbar sind.

Im ersten Endbereichs 5 ist eine Hebelausnehmung 11 vorgesehen, die auf einer dem Mittelteil 3 abgewandten Einführseite 12 und einer der Griffmulde 4 gegenüberliegenden Deckseite 13 des Handgriffs 1 geöffnet ist. An dem der Einführseite 12 zugewandten Ende der Hebelausnehmung 11 sind an Seitenwänden 14, 15 zwei einander gegenüberliegende, sich in Längsrichtung des Handgriffs 1 erstreckende Führungsvorsprünge 16, 17 als Zapfen von Führungsmitteln ausgebildet. An dem der Einführseite 12 abgewandten Ende der Hebelausnehmung 11 sind an den Seitenwänden 14, 15 weiterhin zwei einander gegenüberliegende Anlenkzapfen 18, 19 von Gelenkmitteln ausgebildet, die einen rundlichen Querschnitt aufweisen.

In die Hebelausnehmung 11 mündet an deren der Einführseite 12 abgewandten Ende eine sich durch das Mittelteil 3 und den zweiten Endbereich 6 zu einer Widerlagerseite 20 des Handgriffs 1 erstreckende Zugstangenausnehmung 21 mit rundlichem Querschnitt als Betätigungsstangenausnehmung, in die eine Zugstange 22 als Betätigungsstange einfügbar ist. Die über den wesentlichen Teil ihrer Länge mit einem rundlichen Querschnitt ausgebildete Zugstange 22 weist an einem Widerlagerende 23 einen Gewindeabschnitt 24 auf, auf den eine Widerlagerschraube 25 mit einem verdickten Widerlagerschraubenkopf 26 als Kopf von der Widerlagerseite 20 des Handgriffs 1 aus aufschraubbar ist. An einem dem Widerlagerende 23 gegenüberliegenden Befestigungsende 27 ist die Zugstange 22 mit einem rechteckigen Querschnitt ausgebildet, wobei im endseitigen Endbereich des Befestigungsendes 27 eine sich in Querrichtung erstreckende Stangenachsausnehmung 28 in die Zugstange 22 eingebracht ist.

Der Anlenkhebel 2 des Ausführungsbeispiels gemäß Fig. 1 ist in einem endseitigen Befestigungsabschnitt 29 mit Begrenzungsvorsprüngen 30, 31, 32, 33 der Führungsmittel ausgebildet, die paarweise einander gegenüberliegend an jeweils einer Seitenfläche 34, 35 des Anlenkhebels 2 angeordnet sind. Die Begrenzungsvorsprünge 30, 31, 32, 33 sind länglich als Zapfen ausgebildet und erstrecken sich quer zu der Längsrichtung des Anlenkhebels 2. Die jeweils über eine Seitenfläche 34, 35 vorstehenden Paare von Begrenzungsvorsprüngen 30, 31; 32, 33 weisen im Hinblick auf eine zweckmäßige, fehlerfreie Montage einen Abstand voneinander sowie eine Dicke auf, die im wesentlichen den Dimensionen der Führungsvorsprünge 16, 17 in den beiden Richtungen quer zu der Längsrichtung des Handgriffs 1 entspricht. Im endseitigen Teil des Befestigungsabschnitts 29 des Anlenkhebels 2 ist eine sich aus zwei die endseitigen Begrenzungsvorsprünge 30, 32 querenden Teilabschnitten zusammensetzende Anlenkachsausnehmung 36 eingebracht, wobei zwischen den Teilabschnitten der Anlenkachsausnehmung 36 eine Nut 37 vorgesehen ist, in die der die Stangenachsausnehmung 28 aufweisende Teil der Zugstange 22 unter fluchtender Anordnung der Stangenachsausnehmung 28 sowie der Teilabschnitte der Anlenkachsausnehmung 36 einfügbar ist. Ein sich an den Befestigungsabschnitt 29 anschließender Betätigungsabschnitt 38 des Anlenkhebels 2 ist in ergonomischer Weise gewinkelt ausgeführt und weist eine Länge auf, die wenigstens der Länge des Mittelteils 3 des Handgriffs 1 entspricht.

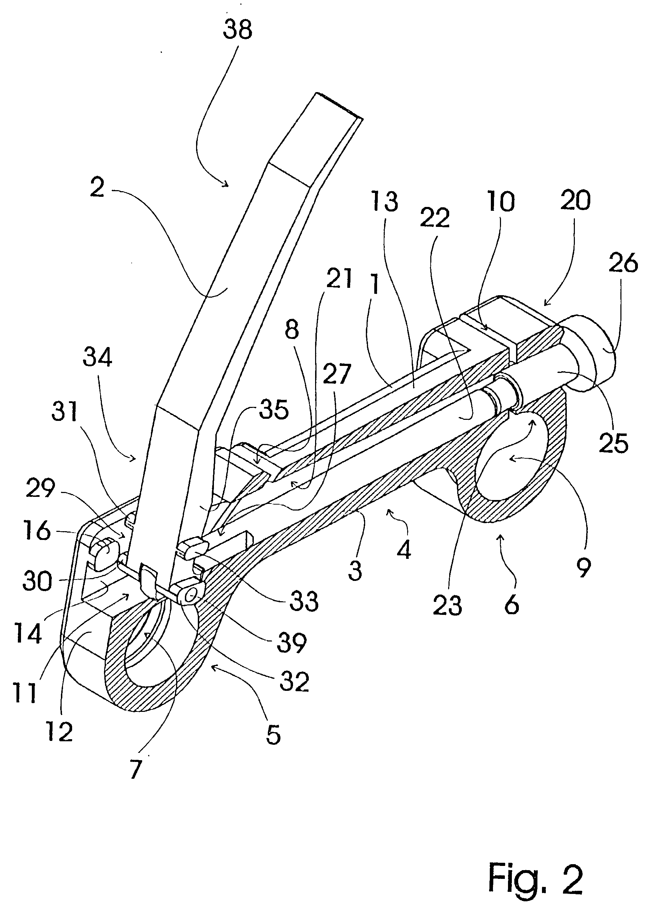

Fig. 2 zeigt in einer teilgeschnittenen perspektivischen Ansicht das Ausführungsbeispiel gemäß Fig. 1 mit dem Handgriff 1 und dem Anlenkhebel 2 in einer Einführstellung. In der Anordnung gemäß Fig. 2 ist der Anlenkhebel 2 mittels einer Verbindungsachse 39 mit der in der Zugstangenausnehmung 21 angeordneten Zugstange 22 verbunden, wobei die Verbindungsachse 39 in die Stangenachsausnehmung 28 und in die Anlenkachsausnehmung 36 eingesteckt sowie die Widerlagerschraube 25 auf den Gewindeabschnitt 24 der Zugstange 22 aufgeschraubt ist. Der Anlenkhebel 2 ist in der Einführstellung nahezu rechtwinklig zu dem Handgriff 1 ausgerichtet ist, so daß der Befestigungsabschnitt 29 des Anlenkhebels 2 von der Einführseite 12 des Handgriffs 1 her in die Hebelausnehmung 11 einfügbar ist. Dabei gleiten die Paare von Begrenzungsvorsprünge 30, 31; 32, 33 an dem jeweiligen Führungsvorsprung 16, 17 vorbei, wobei durch die längliche Ausbildung der Führungsvorsprünge 16,17 sowie der Begrenzungsvorsprünge 30, 31, 32, 33 die rechtwinklige Ausrichtung des Anlenkhebels 2 zu dem Handgriff 1 während des Einführvorgangs sichergestellt ist, bis das der Einführseite 12 abgewandte Ende der Hebelausnehmung 11 erreicht ist.

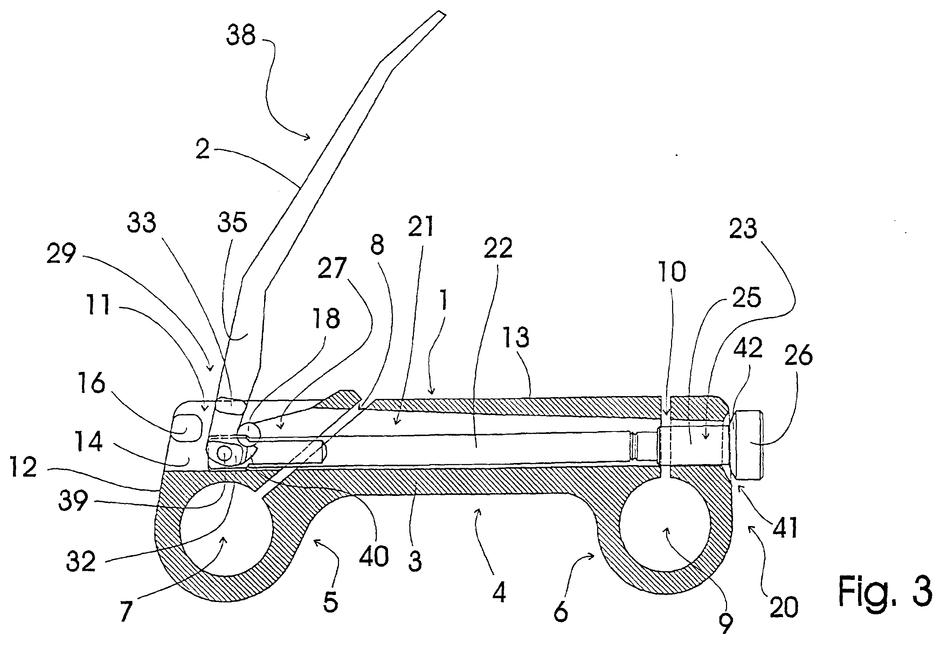

Fig. 3 zeigt in einer teilgeschnittenen Seitenansicht das Ausführungsbeispiel gemäß Fig. 1 mit dem Handgriff 1 und dem Anlenkhebel 2 in der Einführstellung gemäß Fig. 2 am Ende des Einführvorgangs in einer Einführendstellung als erster Stellung, bei dem eine in Verlängerung der endseitigen Begrenzungsvorsprünge 30, 32 an dem Anlenkhebel 2 ausgebildete, hakenartige Anlenknase 40 der Gelenkmittel mit den Anlenkzapfen 18, 19 in Eingriff kommt, wobei die Führungsvorsprünge 16, 17 sowie die Begrenzungsvorsprünge 30, 31, 32, 33 nunmehr freiliegen. Durch die während des Einführvorgangs im wesentlichen gleichbleibende Ausrichtung des Anlenkhebels 2 in bezug auf den Handgriff 1 ist sichergestellt, daß die Anlenknase 40 in der Einführendstellung ordnungsgemäß an den Anlenkzapfen 18, 19 anliegt. In der Einführendstellung weist der Widerlagerschraubenkopf 26 bei bestimmungsgemäßer Justage der Widerlagerschraube 25 nur noch einen verhältnismäßig geringen Abstand von der Widerlagerseite 20 des Handgriffs 1 auf und liegt einem kalottenförmig ausgebildeten Mündungsbereich 41 der Zugstangenausnehmung 21 mit einer zu diesem komplementär ausgebildeten Anlageseite 42 des Widerlagerschraubenkopfs 26 gegenüber.

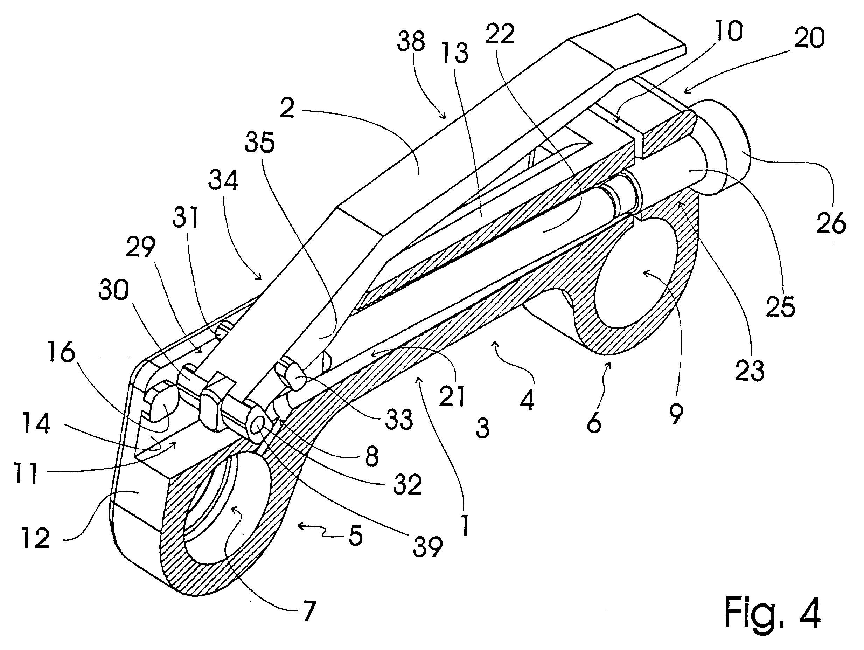

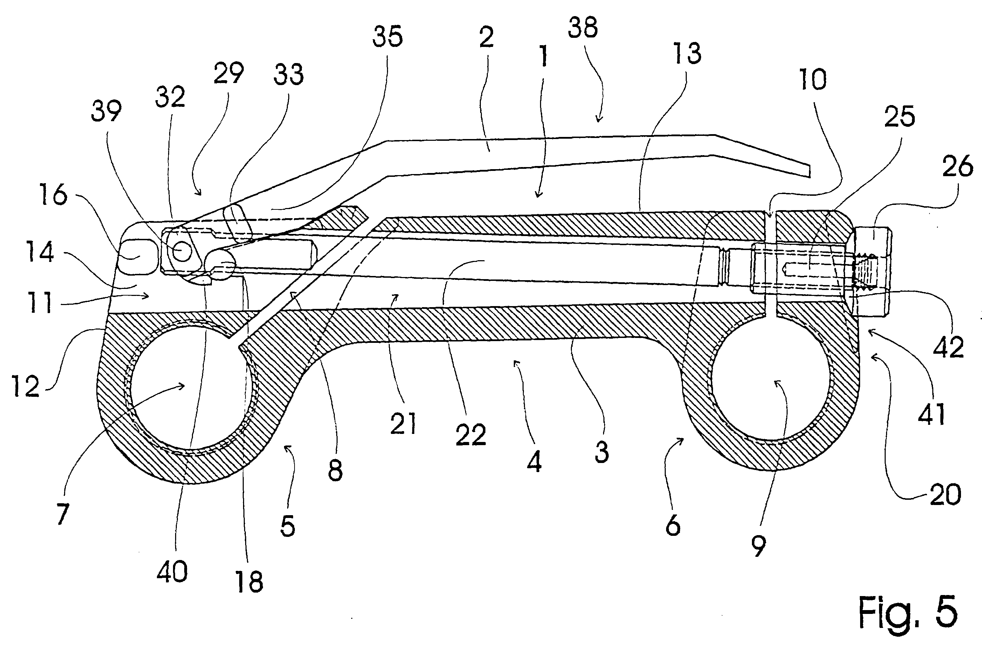

Fig. 4 und Fig. 5 zeigen in einer teilgeschnittenen perspektivischen Ansicht beziehungsweise einer teilgeschnittenen Seitenansicht das Ausführungsbeispiel gemäß Fig. 1 mit dem Handgriff 1 und dem Anlenkhebel 2 in einer Verriegelungsstellung als zweiter Stellung, in der der Anlenkhebel 2 in Richtung des Mittelteils 3 des Handgriffs 1 geklappt ist und im wesentlichen parallel zu diesem verläuft. Bei der Überführung des Anlenkhebels 2 von der in Fig. 2 beziehungsweise Fig. 3 dargestellten Einführendstellung in die Verriegelungsstellung gemäß Fig. 4 beziehungsweise Fig. 5 dreht die mittels der die Führungsvorsprünge 16, 17 sowie die Begrenzungsvorsprünge 30, 31, 32, 33 umfassenden Führungsmittel unter Vermeidung von Beschädigungen bei ansonsten möglicher unsachgemäßer Ausrichtung korrekt angeordnete Anlenknase 40 um die Anlenkzapfen 18, 19, wobei die Zugstange 22 über einen Spannweg in die sich von dem Mündungsbereich 41 an der Widerlagerseite 20 in Richtung der Hebelausnehmung 11 konisch erweiternden Zugstangenausnehmung 21 eingezogen wird. Dabei kommt unter Bewegung der Zugstange 22 in Richtung einer Randseite der Zugstangenausnehmung 21 nach einem Teil des Spannwegs der Widerlagerschraubenkopf 26 mit seiner Anlageseite 42 mit dem Mündungsbereich 41 der Zugstangenausnehmung 21 drehbar zum Anschlag, so daß über den übrigen Teil des Spannwegs die Klemmspalte 8, 10 unter Ausübung einer Zugkraft zwischen dem Handgriff 1 und dem Anlenkhebel 2 sowie Einklemmung von in die Stangenausnehmungen 7, 9 eingeführten Gegenständen verengt werden. Die endseitigen Begrenzungsvorsprünge 30, 32 schließlich kommen in der Verriegelungsstellung zwischen den Führungsvorsprüngen 16, 17 sowie den Anlenkzapfen 18, 19 zum Liegen.

Die Stärke der in den Stangenausnehmungen 7, 9 ausgeübten Klemmkraft ist durch einfache Justage der Widerlagerschraube 25 einstellbar. Bei teilweiser Überführung des Anlenkhebels 2 von der Verriegelungsstellung in die Einführendstellung unter Lockerung der Verklemmung beispielsweise zur Korrektur der Relativstellung der in die Stangenausnehmungen 7, 9 eingeführten Gegenstände gewährleisten die Führungsvorsprünge 16, 17 sowie die Begrenzungsvorsprünge 30, 31, 32, 33 einen anhaltenden Eingriff der Anlenknase 40 mit den Anlenkzapfen 18, 19, auch wenn die durch die Zugstange 22 ausgeübte Zugkraft zwischen dem Handgriff 1 und dem Anlenkhebel 2 nachläßt, so daß ein unbeabsichtigtes Herausgleiten des Anlenkhebels 2 aus der Hebelausnehmung 11 vermieden wird.

## Patentansprüche

1. Vorrichtung zum einstellbaren Verbinden von zwei Gegenständen, insbesondere einer chirurgischen Kopfhalterung mit Anbauteilen an einem Operationstisch, mit einem länglichen Handgriff (1), in dessen beiden Endbereichen (5, 6) jeweils eine Ausnehmung (7, 9) zum Einführen von miteinander zu verbindenden Gegenständen vorgesehen ist, mit einem Anlenkhebel (2), der an einem Ende (29) mit einem Ende (27) einer Betätigungsstange (22) verbindbar ist, wobei das andere Ende (23) der Betätigungsstange (22) an dem Handgriff (1) gegenlagerbar ist, mit an dem Handgriff (1) sowie an dem Anlenkhebel (2) ausgebildeten, voneinander lösbaren Gelenkmitteln (18, 19, 40), mit denen bei Überführung des Anlenkhebels (2) von einer ersten Stellung in eine zweite Stellung auf die Betätigungsstange (22) eine Kraft ausübbar ist, mittels der im Bereich der Ausnehmungen (7, 9) ausgebildete Klemmspalte (8, 10) unter Verklemmung von in die Ausnehmungen (7, 9) eingefügten Gegenständen verengbar sind, und mit an dem Handgriff (1) sowie dem Anlenkhebel (2) ausgebildeten Führungsmitteln (16, 17, 30, 31, 32, 33), mit denen die Gelenkmittel (18, 19, 40) in einer Einführstellung des Anlenkhebels (2) in bezug auf den Handgriff (1) definiert miteinander in Eingriff bringbar sind.

2. Vorrichtung nach Anspruch 1, dadurch gekennzeichnet, daß die Führungsmittel wenigstens zwei paarweise einander gegenüberliegend ausgebildete Führungsvorsprünge (16, 17) umfassen, die an Seitenwänden (14, 15) einer Hebelausnehmung (11) des Handgriffs (1) angeordnet sind.

3. Vorrichtung nach Anspruch 2, dadurch gekennzeichnet, daß die Führungsvorsprünge (16, 17) als sich in Längsrichtung des Handgriffs (1) erstreckende längliche Zapfen ausgebildet sind.

4. Vorrichtung nach Anspruch 2 oder 3, dadurch gekennzeichnet, daß der Abstand der paarweise einander gegenüberliegenden, an verschiedenen Seitenwänden (14, 15) angeordneten Führungsvorsprüngen (16, 17) im wesentlichen der Dicke des Anlenkhebels (2) entspricht.

5. Vorrichtung nach einem der Ansprüche 2 bis 4, dadurch gekennzeichnet, daß die Führungsmittel weiterhin wenigstens ein Paar von einander gegenüberliegenden, jeweils an einer Seitenfläche (34, 35) des Anlenkhebels (2) ausgebildete Begrenzungsvorsprünge (30, 31, 32, 33) umfassen, deren Abstand der Breite der Führungsvorsprünge (16, 17) quer zur Längsrichtung des Handgriffs (1) entspricht oder größer als diese Breite ist.

6. Vorrichtung nach Anspruch 5, dadurch gekennzeichnet, daß jeder Begrenzungsvorsprung (30, 31, 32, 33) als sich in Querrichtung des Anlenkhebels (2) länglich erstreckender Zapfen ausgebildet ist.

7. Vorrichtung nach Anspruch 5 oder 6, dadurch gekennzeichnet, daß der Abstand zwischen einander gegenüberliegenden, an verschiedenen Seitenflächen (34, 35) angeordneten Begrenzungsvorsprüngen (30, 31, 32, 33) der Weite der Hebelausnehmung (11) entspricht.

8. Vorrichtung nach einem der Ansprüche 2 bis 7, dadurch gekennzeichnet, daß die Gelenkmittel zwei einander gegenüberliegende, in der Hebelausnehmung (11) innenseitig der Führungsvorsprünge (16, 17) angeordnete Anlenkzapfen (18, 19) umfassen.

9. Vorrichtung nach Anspruch 8, dadurch gekennzeichnet, daß die Gelenkmittel eine in der ersten Stellung mit den Anlenkzapfen (18, 19) in Eingriff stehende, an dem Anlenkhebel (2) ausgebildete Anlenknase (40) umfassen.

10. Vorrichtung nach einem der Ansprüche 2 bis 9, dadurch gekennzeichnet, daß der Handgriff (1) eine Betätigungsstangenausnehmung (21) aufweist, in der die Betätigungsstange (22) positionierbar ist.

11. Vorrichtung nach Anspruch 10, dadurch gekennzeichnet, daß an dem den Gelenkmitteln (18, 19, 40) gegenüberliegenden Endbereich (6) des Handgriffs (1) ein Mündungsbereich (41) der Betätigungsstangenausnehmung (21) kalottenförmig ausgebildet ist, so daß darin ein abschnittsweise komplementär ausgebildeter Kopf (26) einer auf die Betätigungsstange (22) aufschraubbarer Widerlagerschraube (25) drehbar gelagert ist.
